# EUROPEAN PATENT APPLICATION

(11) **EP 2 669 288 A1**
(43) Date of publication of application: **04.12.2013**
(21) Application number: 12169804.7
(22) Date of filing: 29.05.2012
(51) Int. Cl.: C07F 9/09, C07H 5/06, A61K 31/661, A61K 31/7008, A61P 31/00

(54) **New monosaccharide derivatives and biological applications thereof**

(71) Applicant: LABORATOIRE BIODIM, 75007 Paris (FR)
(72) Inventor: Atamanyuk, Dmytro, 77500 Chelles (FR); Gerusz, Vincent, 75017 Paris (FR)
(74) Representative: Hirsch & Associés

(57) **Abstract**

The invention relates to monosaccharide derivatives of formula (I) 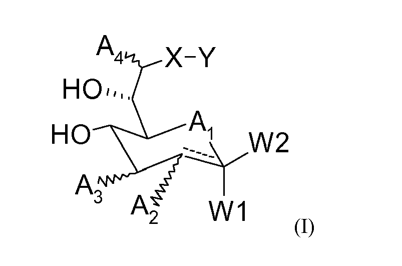 wherein the dotted line represents a possible second bond, X is CH₂, CHF, CF₂, CHOH, O, S or NH, Y is P(O) (ORₐ) (OR_{b}) or P(O) (ORₐ) (NRₐR_{b}), A₁ is O, S, NRₐ, N(CO)Rₐ, N(SO)Rₐ or N(SO₂)Rₐ, A₂ and A₃ are H, F, NR_{c}R_{d}, NR_{c}(CO)R_{d}, NR_{c}(SO)R_{d}, NR_{c}(SO₂)R_{d} and OR_{c} and A₂ may also be an oxime NOR_{c}, A₄ is H, F or OH, W1 and W2 are as defined in the application, as are Rₐ, R_{b}, R_{c} and R_{d}, and their addition salts thereof with acids and bases,
their preparation and their use in the antibacterial prevention and therapy, used alone or in association with antibacterials, antivirulence agents or drugs reinforcing the host innate immunity,
and pharmaceutical compositions and associations containing them.

## Description

The invention relates to new monosaccharide derivatives, their preparation and intermediates, their use as drugs and pharmaceutical compositions containing them.

The invention also relates to new monosaccharide derivatives capable of inhibiting bacterial heptose biosynthesis and thereby lowering or suppressing bacterial virulence, as well as their antibacterial pharmaceutical applications in preventive or curative treatment or in combination therapy.

The invention particularly relates to new monosaccharide derivatives capable of inhibiting the GmhA and/or HldE enzymes of bacterial heptose synthesis, thereby lowering or suppressing bacterial virulence, as well as their antibacterial pharmaceutical applications.

The lipopolysaccharide (LPS) is a major component of the outer membrane of Gram-negative bacteria. It is composed of three regions: the lipid A, the core oligosaccharide and the O antigen. The core oligosaccharide is divided into the inner core and the outer core. The inner core consists in a motif of five sugars: two Kdo (Kdo: 3-deoxy-D-manno-octulosonic acid) and three successive heptoses. The first heptose transfer is catalysed by the Heptosyltransferase I (protein WaaC) and the second heptose transfer by the Heptosyltransferase II (protein WaaF). The natural donor substrate of these transferases is ADP heptose, which is synthesized in bacteria from sedoheptulose-7-phosphate by the successive enzymatic steps catalyzed by the following enzymes: GmhA, HldE-K (former or other nomenclature: RfaE-K), GmhB, HldE-AT (former or other nomenclature: RfaE-AT) and HldD (former or other nomenclature: RfaD, WaaD) (Journal of Bacteriology, 2002, 184, 363).

Heptose synthetic pathway is conserved among Gram negative bacterial species and is necessary for full LPS synthesis. It has been demonstrated that a complete LPS is necessary for Gram negative bacterial pathogenesis. Bacteria lacking heptoses display a so-called "deep-rough phenotype" due to the absence of the outer core and the O-antigen. While still able to survive as the commensal flora, they are unable to yield a productive infection in the host and are very sensitive to detergents or hydrophobic antibiotics as well as to the bactericidal effect of the host complement (Annu. Rev. Biochem. 2002, 635).

By preventing full LPS development in Gram negative bacteria, inhibitors of bacterial GmhA and/or HldE-K would be expected to induce a high sensitivity to the host complement and therefore be able to prevent or inhibit bacterial infection. Such inhibitors would provide a novel way to treat or prevent bloodstream infections caused by pathogenic Gram negative bacteria, without affecting the commensal flora and with less selective pressure than conventional antibacterial agents.

A few inhibitors of bacterial heptose synthesis have been reported in the literature, targeting GmhA (Chem. Eur. J. 2011, 11305), HldE (Chem. Biol. 2006, 437; WO2006058796; WO2008038136; Bioorg. Med. Chem. 2009, 1276; WO2010001220) and Waac/Waaf (Bioorg. Med. Chem. Lett. 2008, 4022; Chem. Eur. J. 2008, 9530). However, no results on the bacterial inhibition of the LPS biosynthesis have been reported yet for these inhibitors, raising concern on their ability to reach their cytosolic target at an effective concentration. Thus, despite their attractiveness, these bacterial targets are still largely unexploited at this time since there are no drugs on market or on advanced clinical phases. One of the purposes of the present invention is therefore to provide novel compounds active on these targets while also demonstrating the ability to inhibit LPS formation on clinically relevant Gram-negative bacteria.

Finally, JACS 1998, 120, 11027 describes the compound 3-Deoxy-D-altro-octulosonate 8-phosphate, prepared as a mechanistic proof, in order to demonstrate the activity of an enzyme involved in phenylalanine biosynthesis, with no direct therapeutic target.

The invention relates to new compounds having the general formula (I) 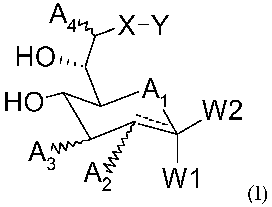 wherein,
- the dotted line represents a possible second bond;
- X is CH₂, CHF, CF₂, CHOH, O, S or NH;
- Y is P(O)(ORₐ)(OR_{b}) or P(O)(ORₐ)(NRₐR_{b});
- A₁ is O, S, NRₐ, N(CO)Rₐ, N(SO)Rₐ or N(SO₂)Rₐ;
- A₂ and A₃, identical or different, are H, F, NR_{c}R_{d}, NR_{c}(CO)R_{d}, NR_{c}(SO)R_{d}, NR_{c}(SO₂)R_{d} or OR_{c}, and A₂ may also be an oxime NOR_{c}, in which case the dotted line doesn't represent a bond;
- A₄ is H, F or OH;
- W1 and W2 identical or different, are selected from the group consisting of H, F, CN, (C₁-C₆)alkyl, (C₁-C₆)fluoroalkyl, (C₂-C₆)alkenyl, (C₂-C₆)alkynyl, OR_{c}, (CO)OR_{c}, (CO)NR_{c}R_{d}, NR_{c}R_{d}, O(CO)R_{c}, O(CO)OR_{c}, O(CO)NR_{c}R_{d}, S(O)R_{c}, S(O)₂R_{c}, (C₁-C₆) alkyl-OR_{c}, (C₁-C₆)alkyl-NR_{c}R_{d}, (C₁-C₆)alkyl-(CO)OR_{c}, (C₁-C₆)alkyl-(CO)NR_{c}R_{d}, (C₁-C₆)alkyl-O(CO)R_{c}, (C₁-C₆) alkyl-O(CO)OR_{c}, (C₁-C₆)alkyl-O(CO)NR_{c}R_{d}, (C₁-C₆)alkyl-S(O)R_{c} or (C₁-C₆)alkyl-S(O)₂R_{c}, NR_{c}(CO)R_{d}, NR_{c}(CO)OR_{d}, NR_{c}(SO₂)R_{d};
   or W1 and W2 form together a saturated 3-6 membered carbon cycle or heterocycle with 1 to 3 ring heteroatoms selected from N, O and S;
   W2 being absent when the dotted line represents a second bond;
- Rₐ and R_{b}, identical or different, are selected from the group consisting of H, (C₁-C₆)alkyl, C₁-C₆)fluoroalkyl, (C₁-C₆)alkyl-OH, (C₁-C₆)alkyl-O-(C₁-C₆)alkyl;
- R_{c} and R_{d}, identical or different, are selected from the group consisting of H, (C₁-C₆)alkyl, C₁-C₆)fluoroalkyl, (C₂-C₆)alkenyl, (C₂-C₆)alkynyl, (C₁-C₆)alkyl-OH, (C₁-C₆)alkyl-O-(C₁-C₆)alkyl, Ph, Het, CH₂Ph, CHMePh, CMe₂Ph, CH₂Het, CHMeHet, CMe₂Het, Het being a 4-6 membered saturated or unsaturated heterocycle with 1 to 3 ring heteroatoms selected from N, O and S and optionally substituted by a keto, alkyl, fluoroalkyl, OH, NH2, O-alkyl, NH-alkyl or N(dialkyl), the above alkyls being C₁-C₆;
   with the proviso that when A₂ and A₃ are OH and A₄ is H then A₁ may not be O;
   and to the exclusion of 3-Deoxy-D-altro-octulosonate 8-phosphate;
   and their addition salts thereof with acids and bases.

According to a preferred embodiment, Y is P(O)(OH)_{2·}

According to another preferred embodiment, X is CH₂, CHF, CF₂, CHOH or O.

According to another preferred embodiment, A₁ is O.

According to another preferred embodiment, A₃ is H, F, OR_{c} or NR_{c}R_{d}.

According to another preferred embodiment, W1 and W2, identical or different, are selected from the group consisting of H, (C₁-C₆)alkyl, (C₁-C₆)fluoroalkyl, OR_{c} and (C₁-C₆)alkyl-OR_{c}.

According to another preferred embodiment, R_{c} and R_{d} are selected from the group consisting of H, (C₁-C₆)alkyl, (C₁-C₆)fluoroalkyl, (C₁-C₆)alkyl-OH, (C₁-C₆)alkyl-O-(C₁-C₆)alkyl and Het.

Among the acid salts of the products of formula (I), there may be cited, among others, those formed with mineral acids, such as hydrochloric, hydrobromic, hydroiodic, sulfuric or phosphoric acid or with organic acids such as formic, acetic, trifluoroacetic, propionic, benzoic, maleic, fumaric, succinic, tartaric, citric, oxalic, glyoxylic, aspartic, alkanesulfonic acids, such as methanesulfonic and ethanesulfonic acids, arylsulfonic acids such as benzenesulfonic and para-toluenesulfonic acids.

Among the alkaline salts of the products of formula (I), there may be cited, among others, those formed with mineral alkalis such as, for example, sodium, potassium, lithium, calcium, magnesium or ammonium or organic bases such as, for example, methylamine, ethylamine, propylamine, trimethylamine, diethylamine, triethylamine, N,N-dimethylethanolamine, tris(hydroxymethyl)aminomethane, ethanolamine, pyridine, piperidine, piperazine, picoline, dicyclohexylamine, morpholine, benzylamine, procaine, lysine, arginine, histidine, N-methylglucamine.

In the general formula (I), as applied herein:
"(C₁-C₆)alkyl" means any linear, branched, mono or bicyclic hydrocarbon groups comprising 1 (or 3 for a cycle) to 6 carbon atoms, preferably methyl, ethyl, n-propyl, isopropyl, n-butyl, isobutyl and t-butyl, n-pentyl, isopentyl, neopentyl, cyclopropyl, cyclobutyl, cyclopentyl, cyclohexyl, bicyclo[3.1.0]hexane, bicyclo[2.2.0]hexane, spiro[2.2]pentane, spiro[2.3]hexane or means an alkyl chain including or substituted by a small cycloalkyl, itself possibly substituted by an alkyl, or means a small cycloalkyl substituted by alkyl;
"(C₂-C₆)alkenyl" and "(C₂-C₆)alkynyl" as applied herein means any linear, branched or cyclic hydrocarbon groups of 2 to 6 carbon atoms, having at least one double bond or one triple bond and preferably ethenyl, propenyl, butenyl, cyclohexenyl, ethynyl, propargyl, butynyl;
"Ph" means Phenyl.

Among the cycles which may be formed by W1 and W2 there may be cited the following non-limiting examples: (C₃-C₆)cycloalkyl, oxetane, azetidine, thietane 1,1-dioxide, tetrahydrofurane, tetrahydrofuranone, tetrahydropyrane, tetrahydropyranone, pyrolidine, pyrolidinone, piperidine, piperidinone, oxazolidinone, isoxasolidine, imidazolidinone, dioxolane, 1,4-dioxane, piperazine, piperazinone, piperazinedione, morpholine, [1,2,5]thiadiazolidine 1,1-dioxide, [1,2,3]oxathiazolidine 2,2-dioxide, [1,3]oxazinan-2-one, azepanone.

Among the compounds of the invention, there may be cited the following compounds:
7-O-phosphate-D-*glycero*-D-*gluco*-2-deoxy-2-amino-1-deoxy-heptopyranoside,
7-O-phosphate-D-*glycero*-D-*gluco*-2-deoxy-2-amino-heptopyranoside,
and their salts, in particular their hydrochlorides.

The compounds of formula I may be prepared by any processes known to be applicable to the preparation of chemically related compounds (for a review example: Curr. Org. Chem. 2008, 1021). Such processes may use known starting materials or intermediates which may be obtained by standard procedures of organic chemistry. The following processes provide a variety of non-limiting routes for the production of the compounds of formula I and their intermediates.

Examples of processes to prepare compounds of formula (I) and salts thereof include in non-limiting manner the transformation into compounds of formula (I) of compounds of formula (II): 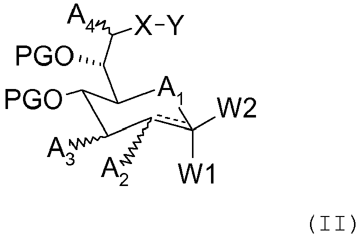 wherein X, Y, A₁, A₂, A₃, A₄, W1 and W2 are as above defined, all of these groups being optionally protected by one or several identical or different group PG, PG being H or an appropriate identical or different protecting group (non-limiting examples include optionally substituted benzyl, silyl, acyl, carboxybenzyl and benzoyl);
by one or more of the non-limiting appropriate following reactions, performed in an appropriate order, to achieve the desired transformations on W and/or X and/or Y and/or A and/or PG defined above:
protection of reactive functions,
deprotection of reactive functions,
halogenation,
halogenoacylation,
halogenohydroxylation,
epoxide formation and opening,
metathesis,
dehalogenation,
dealkylation,
alkylation of amine, aniline, alcohol and phenol, oxidation,
Wittig type reaction on carbonyl groups,
dihydroxylation reaction of carbon-carbon double bonds,
reduction of nitro, esters, cyano, aldehydes, thioethers, double and triple bonds,
transition metal-catalyzed reactions,
etherification,
acylation,
sulfonylation/introduction of sulfonyl groups,
saponification/hydrolysis of esters groups,
halogen exchange,
nucleophilic substitution with amine, thiol or alcohol,
reductive amination,
phosphorylation,
sulphatation,
phosphitation,
phosphonylation,
amidation,
phosphoramidation,
oxime formation via addition of an hydroxylamine to a keto group or via nitroglycal reduction,
introduction of Rₐ, R_{b}, R_{c}, R_{d} groups on A₁, A₂, A₃, Y, W₁ or W₂ groups;
salification;
all of these reactions optionally followed by deprotection of PG to hydrogen.

All these reactions are classical and known to the skilled chemist. General and more specific references can be cited, including, as a general reference, Michael B. Smith, Jerry March, March's Advanced Organic Chemistry: Reactions, Mechanisms, and Structure, 6th Edition, Wiley, 2007, and as more specific references, in particular those listed hereafter:
Protection and deprotection of reactive functions:
   Peter G. M. Wuts, Theodora W. Greene, Greene's Protective Groups in Organic Synthesis, 4th Edition, Wiley, 2006);
   Halogenation reactions: Hanessian, Preparative Carbohydrate Chemistry, CRC Press, 1997;
   Halogenoacylation reactions: Journal of Carbohydrate Chemistry 2007, 26, 141;
   Halogenohydroxylation: Journal of Carbohydrate Chemistry 2001, 20, 359;
   Epoxide formation and opening: J. Org. Chem., 1993, 58, 3761 ; Tet.Lett. 1994, 35, 8433;
   Halogenophosphorylation reactions: Chem. Eur. J. 2008, 14, 9530;
   Metathesis reaction: Tet. Lett. 2011, 52, 6767;
   Oxidation reaction: Chem. Eur. J., 2010, 16, 8545;
   Oxime formation via addition of an hydroxylamine to a keto group: Carbohydr. Res. 2009, 344, 2127; Carbohydr. Res. 1999, 320, 250; Tetrahedron 2001, 57, 7919; Tetrahedron: Asymmetry 2011, 22, 109; or via nitroglycal reduction: Eur. J. Org. Chem. 2010, 3579;
   Wittig type reaction on carbonyl groups and homologations by Grignard reagents: J. Org. Chem. 2000, 65, 6493; Chem. Eur. J. 2008, 14, 9530 ; Pol. J. Chem. 1996, 70, 45; Angew. Chem. 2008, 120, 1731; Carbohydr. Res. 2005, 340, 2808; Carbohydr. Res. 1986, 152, 329; J. Am. Chem. Soc. 2006, 128, 8078; Tet. Lett. 2011, 52, 6767; Carbohydr. Res. 2001, 332, 225;
   Dihydroxylation reaction of carbon-carbon double bonds: Noe, M. C., Letavic, M. A., Snow, S. L. 2005.and asymmetric dihydroxylation of alkenes: Organic Reactions. 109-625;
   Transition metal-catalyzed reactions: Matthias Beller, Carsten Bolm, Transition Metals for Organic Synthesis, Wiley, 2004;
   Phosphorylation reaction: Chem. Eur. J. 2011, 17, 11305 - 11313; Carbohydrate Research 2005, 340, 2808; Carbohydrate Research 2003, 338, 2571, Tetrahedron Letters 1999, 40, 1869; Org. Lett. 2001, 3, 2009; J. Org. Chem. 2000, 65, 4498, Eur. J. Org. Chem. 2000, 3433;
   Introduction of Ra, Rb, Rc, Rd groups on A1, A2, A3, Y, W1 or W2 groups are performed by known alkylation, acylation, sulfoxidation, sulfonylation or oxime formation reactions.

Compounds of formula (I) and salts thereof may also be prepared in non-limiting manner by transformation at the anomeric position of compounds of formula (III), or a salt thereof: 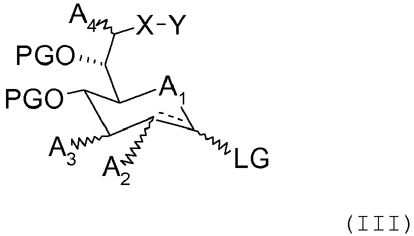 wherein A₁, A₂, A₃, A₄, X and Y are as above defined, optionally protected by one or several identical or different protecting group PG as defined above, LG is an appropriate leaving group (non-limiting examples include hydroxyl, thioaryl, O-acyl, halogen, phosphonium, sulfonyloxy, NR_{c}R_{d} or OR_{c}) .
Displacement of the leaving group at the anomeric position of compounds of formula (III) occurs by optional leaving group activation with an halogenated reagent (non-limiting example include NCS or NBS in the case of thioaryl), following nucleophilic substitution with any appropriate nucleophile (non-limiting examples include allyltrimethylsilane with appropriate Lewis acid(s) in the case of allylation of acetate leaving group, or with DAST in the case of the fluoration of the hydroxyl leaving group), following with optional hydrolysis, alkylation, acylation, reduction, oxidation, substitution, optionally followed by deprotection of PG to hydrogen.

Compounds of formula (I) and salts thereof may also be prepared in non-limiting manner by transformation at the anomeric oxygen from compounds of formula (IV), or a salt thereof: 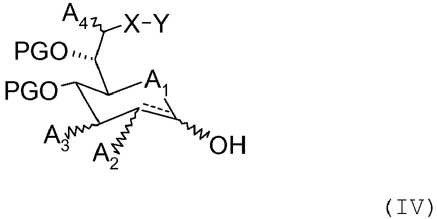 wherein A₁, A₂, A₃, A₄, X and Y are as above defined, optionally protected by one or several identical or different protecting group PG as defined above,
Nucleophilic substitution by the anomeric hydroxyl with any appropriate electrophilic reacting groups optionally attached to a leaving group LG as defined above may achieve the desired transformation (non-limiting example includes iodomethane with appropriate base like silver oxide in the case of a methylation), optionally followed by deprotection of PG to hydrogen;

Compounds of formula (I) and salts thereof or intermediates of the synthetic route towards compounds of formula (I) may also be obtained in non-limiting manner by transformation of compounds of formula (V) or a salt thereof, by reacting a compound of formula (V): 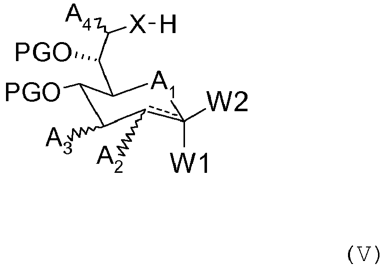 with Y-LG,
X, Y, A₁, A₂, A₃, A₄, W1, W2 and LG defined as above with X, Y, A₁, A₂, A₃, A₄, W1 and W2 optionally protected by one or several identical or different protecting groups PG defined as above;
(non-limiting example includes phosphorylation with (RₐO)(R_{b}O)P(O)-LG, such as nucleophilic substitution in case LG is halogen, or Mitsunobu reaction in case LG is hydroxy), followed by optional oxidation (non-limiting example includes mCPBA or DDQ oxidation of phosphite to phosphate derivatives), optionally followed by deprotection of PG to hydrogen;

Compounds of formula (I) and salts thereof or intermediates of the synthetic route towards compounds of formula (I) may also be obtained in non-limiting manner by transformation of compounds of formula (VI) or a salt thereof, by reacting a compound of formula (VI): 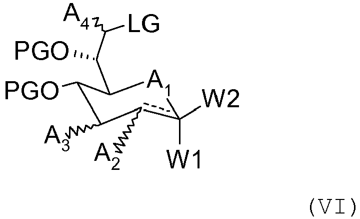 with MeP(O)(ORₐ)(OR_{b}), MeP(O)(ORₐ)(NRₐR_{b}),
CHFP(O)(ORₐ)(OR_{b}), CHFP(O)(ORₐ)(NRₐR_{b}), CF₂P(O)(ORₐ)(OR_{b}) or CF₂P(O)(ORₐ)(NRₐR_{b}),
in the presence of a suitable base,
X, Y, A₁, A₂, A₃, A₄, Rₐ, R_{b}, W1, W2 and LG defined as above and optionally protected by one or several identical or different protecting groups PG as defined above; (non-limiting example includes methylphosphonylation, fluoromethylphosphonylation or difluoromethylphosphonylation with bases such as BuLi or LDA), optionally followed by deprotection of PG to hydrogen;

Compounds of formula (I) and salts thereof or intermediates of the synthetic route towards compounds of formula (I) may also be obtained in non-limiting manner by deprotection of compounds of formula (VII) or a salt thereof: 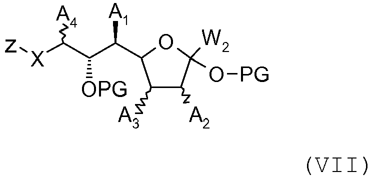 Z defined as Y or PG;
X, Y, A₁, A₂, A₃, A₄ and W2 defined as above and optionally protected by one or several identical or different protecting groups PG as defined above; in the presence of suitable deprotection conditions (non-limiting example includes debenzylation and/or debenzoylation under hydrogenation conditions according to Carbohydr. Res. 2005, 340, 2808);

Compounds of formula (I), salts thereof, and intermediates of the synthetic route towards compounds of formula (I) may also be obtained by homologation of corresponding pentoses or hexoses according to known processes described for example in J. Org. Chem. 2000, 65, 6493; Chem. Eur. J. 2008, 14, 9530 ; Pol. J. Chem. 1996, 70, 45; Angew. Chem. 2008, 120, 1731; Carbohydr. Res. 2005, 340, 2808; Carbohydr. Res. 1986, 152, 329; J. Am. Chem. Soc. 2006, 128, 8078; Tet. Lett. 2011, 52, 6767; Carbohydr. Res. 2001, 332, 225.

Compounds of formula (I) are capable of inhibiting bacterial heptose synthesis which makes them useful as drugs for preventing or treating bacterial infections and another object of the invention is the use of the compounds of formula (I) as drugs.
The drugs of the invention are especially useful for the prevention and therapeutical treatment of severe infections due to Gram-negative bacteria able to disseminate in blood such as the non-limiting following species (spp.): *Escherichia coli, Enterobacter, Salmonella, Shigella, Pseudomonas, Burkholderia, Acinetobacter, Neisseria, Klebsiella, Serratia, Citrobacter, Proteus, Yersinia, Haemophilus, Legionella, Moraxella* and *Helicobacter pylori.*

The invention also relates to pharmaceutical compositions comprising an effective amount of at least one compound of formula (I) such as above defined, in association with a pharmaceutically acceptable carrier.

Said pharmaceutical compositions are advantageously formulated to be administered under topic, oral, parenteral, and preferably injectable routes, with individual doses appropriate for the patient to be treated.

The compositions according to the invention can be solid or liquid and be present in the pharmaceutical forms commonly used in human medicine, such as for example, plain or sugar-coated tablets, gelatin capsules, granules, suppositories, inhalation spray, injectable preparations, ointments, creams, gels; they are prepared according to the customary methods. The active ingredient(s) can be incorporated in same, using excipients which are customarily used in these pharmaceutical compositions, such as talc, gum arabic, lactose, starch, magnesium stearate, cocoa butter, aqueous or non-aqueous vehicles, fatty substances of animal or vegetable origin, paraffin derivatives, glycols, various wetting agents, dispersants or emulsifiers, preservatives.

These compositions can in particular be present in the form of a powder intended to be dissolved extemporaneously in an appropriate vehicle, for example, non-pyrogenic sterile water.

The dose administered varies according to the condition treated, the patient in question, the administration route and the product envisaged. It can, for example, be comprised between 0.01 g and 10 g per day, by oral route in humans or by intramuscular or intravenous route.

The drugs according to the invention can also be used, for the prevention as for the treatment of bacterial infections, in the form of associations with antibacterials or antivirulence agents or drugs reinforcing the host innate immunity. Of particular interest is the synergy obtained by the association with lipophilic compounds that have difficulties crossing the full hydrophilic LPS membrane, such as macrolides, streptogramins, pleuromutilins, FabI inhibitors, rifamycins and lipopeptides. Also of interest is the association with GM-CSF (Granulocyte macrophage colony-stimulating factor), an approved white blood cell growth factor.

A further object of the invention is therefore the associations of the compounds of formula (I) with antibacterials and/or antivirulence agents and/or drugs reinforcing the host innate immunity and, in particular with macrolides or streptogramins or pleuromutilins or FabI inhibitors, rifamycins or lipopeptides or GM-CSF as well as pharmaceutical compositions containing such associations.

Illustrations of the invention are given in the following examples.

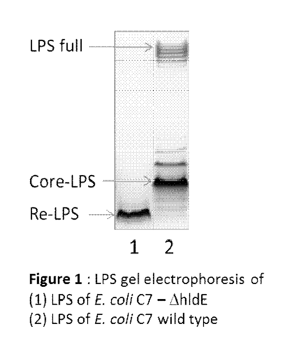

In the results concerning the pharmacological study of the compounds of the invention, it is referred to Figure 1, which provides positive and negative controls obtained with a gel electrophoresis of (1) LPS of *E.coli* C7-ΔhldE and (2) LPS of *E.coli* C7 wild type.

### Experimental part

**Materials and procedures -** All chemicals are commercially available unless indicated otherwise and were used without further purification. Proton nuclear magnetic resonance (NMR) spectra were recorded on either a 300 or 400 MHz Brüker instrument, and chemical shifts are reported in parts per million downfield from the internal standard tetramethylsilane (TMS). Abbreviations for NMR data are as follows: s=singlet, d=doublet, t=triplet, q=quadruplet, quint=quintuplet, sext=sextuplet, m=multiplet, dd=doublet of doublets, dt=doublet of triplets, td=triplet of doublets, tt=triplet of triplets, br=broad. J indicates the NMR coupling constant measured in Hertz. CDCl₃ is deuteriochloroform, DMSO-d₆ is hexadeuteriodimethylsulfoxide, and CD₃OD is tetradeuteriomethanol. Mass spectra were obtained using electrospray ionization (ESI) techniques on an Agilent 1100 Series LCMS and 2795 Alliance Waters LCMS. Analtech Silica Gel GF and E. Merck Silica Gel 60 F-254 thin layer plates were used for thin layer chromatography. Flash chromatography was carried out on Flashsmart Pack cartridge irregular silica 40-60µm or spherical silica 20-40µm. Preparative thin layer chromatography was carried out on Analtech Silica Gel GF 1000 µm 20x20 cm.

The meaning of certain abbreviations is given herein. ESI refers to electrospray ionization, HPLC refers to high pressure liquid chromatography, LCMS refers to liquid chromatography coupled with a mass spectrometer, M in the context of mass spectrometry refers to the molecular weight, MS refers to mass spectrometer, NMR refers to nuclear magnetic resonance, pH refers to potential of hydrogen, TFA refers to trifluoroacetic acid, TEA refers to triethylamine, DIPEA refers to *N,N-*diisopropylethylamine, HOBt refers to 1-hydroxybenzotriazole, THF refers to tetrahydrofuran, DCM refers to dichloromethane, ACN or MeCN refers to acetonitrile, EtOAc refers to ethyl acetate, DME refers to 1,2-dimethoxyethane, DMF refers to *N,N-*dimethylformamide, DEAD refers to diethylazodicarboxylate, AIBN refers to 2,2'-Azobis(2-methylpropionitrile), EDC, EDCI or EDAC refers N-(3-dimethylaminopropyl)-N'-ethylcarbodiimide hydrochloride, DMAP or 4-DMAP refers to 4-(dimethylamino)pyridine, Me refers to methyl, Et refers to ethyl, Bu refers to butyl, dppf refers to 1,1'-bis(diphenylphosphino)ferrocene, DBU refers to 1,8-diazabicyclo[5.4.0]undec-7-ene, TBS or TBDMS refers to tetrabutyldimethylsilyl, TBDPS refers to tetrabutyldiphenylsilyl, Ac refers to acetyl, Ph refers to phenyl, Bn refers to benzyl, Cbz refers to benzyl chloroformate, DIPA refers to diisopropylamine, NBS refers to N-bromosuccinimide, DMSO refers to dimethylsulfoxide, TLC refers to thin layer chromatography.

### Example 1: 7-O-phosphate-D-glycero-D-gluco-2-deoxy-2-amino-1-deoxy-heptopyranoside hydrochloride

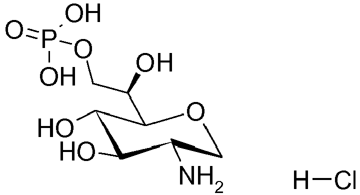

### Step 1: 2-((6S,7S,8aS)-6-Ethylsulfanyl-8-hydroxy-2-phenyl-hexahydro-pyrano[3,2-d][1,3]dioxin-7-yl)-isoindole-1,3-dione

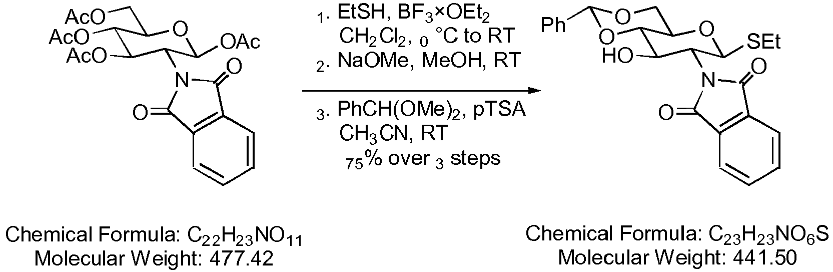

To a solution of 1,3,4,6-tetra-O-acetyl-2-deoxy-2-phthalimido-β-D-glucopyranoside (126 g, 0.26 mol) in anhydrous CH₂Cl₂ (1100 mL) was added 4Å molecular sieves (50 g, ground, activated) and the suspension stirred for 15 minutes at room temperature, EtSH (25.4 mL, 0.34 mol) was charged and the reaction mixture was cooled to 0 °C. A solution of BF₃·OEt₂ (42 mL, 0.34 mol) in anhydrous CH₂Cl₂ (100 mL) was added dropwise and the mixture allowed to gradually warm up to room temperature over 16 h. The progress of the reaction was monitored by TLC (petrol-acetone 7:3) and required additional amount of EtSH (25.4 mL, 0.34 mol) and BF₃·OEt₂ (added neat, 42 mL, 0.34 mol), charged at room temperature. The reaction was warmed at 40 °C for 3 h, until TLC indicated completion of the reaction. The mixture was filtered and a sat. aq. solution of NaHCO₃ followed by solid NaHCO₃ were added upon vigorous stirring until evolution of CO₂ ceased. The layers were separated and the organic layer was dried (MgSO₄), filtered and concentrated *in vacuo.* The crude mixture was then dissolved in MeOH (700 mL) and NaOMe (25% in MeOH) added to pH 10. The reaction was complete within 2 h as judged by TLC (toluene-acetone 1:1) and was neutralised with Amberlyst®15 acidic resin, filtered and concentrated. The resulting white solid was dissolved in 1000 mL of anhydrous CH₃CN and PhCH(OMe)₂ was added (90 mL, 0.60 mol), followed by p-toluenesulfonic acid (9.9 g, 0.05 mol, pH of the mixture = 3) at room temperature. After 2 h an additional portion of PhCH(OMe)₂ (10 mL, 0.07 mol) was added and the reaction was allowed to stir at room temperature for a further 30 minutes, until all the starting material was consumed, as judged by TLC (toluene-acetone 1:1 and 4:1). After neutralisation with Et₃N (approx. 10 mL), the solution was concentrated *in vacuo.* The crude reaction mixture was dissolved in 700 mL of CH₂Cl₂ and washed with 250 mL of sat. aq. NaHCO₃. The layers were separated and the aqueous layer was extracted with CH₂Cl₂(2 × 100 mL). The combined organic layers were dried (MgSO₄) filtered and concentrated *in vacuo.* The residue was crystallised from AcOEt-Petrol (approximately 2:1 mixture) to give 84.8 g (0.19 mol, 74% over 3 steps) of the benzylidine acetal protected title sugar as a white solid.
MS (ESI+) m/z 442 (M+H)⁺.

### Step 2: 2-((6S,7S,8aS)-8-Benzyloxy-6-ethylsulfanyl-2-phenyl-hexahydro-pyrano[3,2-d][1,3]dioxin-7-yl)-isoindole-1,3-dione

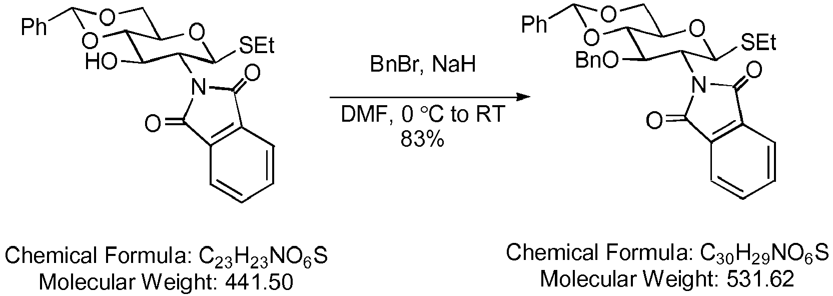

A solution of previously prepared sugar (39.6 g, 0.09 mol) and BnBr (22.4 mL, 0.19 mol) in 150 mL of DMF (DMF stored over 4Å MS, water content <0.01% by Karl Fisher) was added dropwise (over 25 min) to a stirred suspension of NaH (60% in paraffin, 5.4 g, 0.13 mol) at 0 °C. After 1 h the cooling bath was removed and the reaction allowed to stir at room temperature for 1 h, then quenched with 10 mL of MeOH, diluted with 500 mL of AcOEt and washed with 400 mL of 0.5M HCl. The aqueous layer was extracted with AcOEt (2x100 mL) and the combined organic layers were washed with 200 mL of sat. aq. NaHCO₃ followed by 200 mL of brine, then dried over Na₂SO₄, filtered and the solvent was removed *in vacuo.* The residue was purified by column chromatography (SiO₂, toluene-acetone) to give the fully protected title compound as a viscous oil (39.5 g, 83% yield).
MS (ESI+) m/z 532 (M+H)⁺.

### Step 3: 2-((2S,3S,5S)-4,5-Bis-benzyloxy-2-ethylsulfanyl-6-hydroxymethyl-tetrahydro-pyran-3-yl)-isoindole-1,3-dione

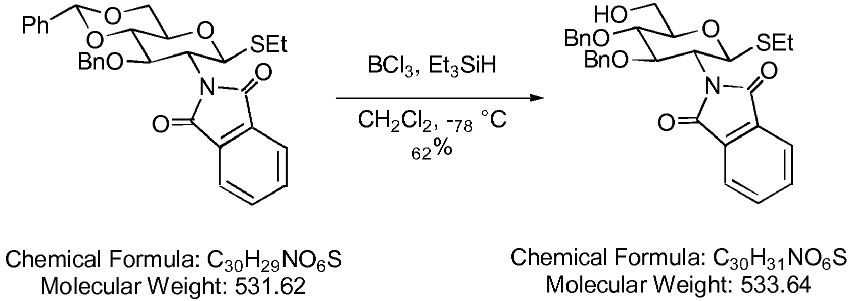

To a solution of previously prepared sugar (17.6 g, 33.1 mmol) in anhydrous CH₂Cl₂ (480 mL) were added 4Å molecular sieves (17 g, ground, activated) and the resulting suspension was stirred at room temperature for 20 min, then cooled down to -78 °C. Et₃SiH (15.8 mL, 98.9 mmol) was added followed by a dropwise addition (over 15 min) of BCl₃ solution (1M solution in CH₂Cl₂, 112 mL, 112 mmol). The reaction (monitored by TLC, petrol-acetone, 7:3) was complete within 20 minutes and then quenched with Et₃N (100 mL) and filtered. The solution was washed with 200 mL of NaHCO₃ (5% aqueous solution) and the aqueous layer was extracted with 200 mL of CH₂Cl₂. The combined organic layers were dried over MgSO₄, filtered and concentrated *in vacuo.* The residue was crystallised from MeOH to give compound 5 as white needles (1^{st} crop of crystals 6.43 g, 2^{nd} crop 4.13 g, total yield 62%).
MS (ESI+) m/z 534 (M+H)⁺.

### Step 4: 2-((2S,3S,5R)-4,5-Bis-benzyloxy-2-ethylsulfanyl-6-vinyl-tetrahydro-pyran-3-yl)-isoindole-1,3-dione

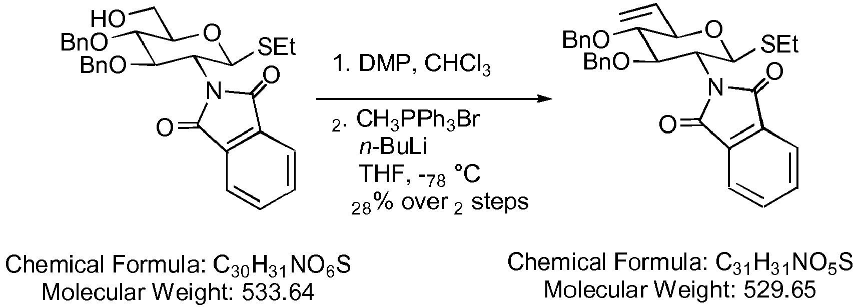

To a room temperature solution of previously prepared sugar (3.4 g, 6.37 mmol) in CHCl₃ (50 mL) was added Dess-Martin periodinane in two portions (3.7 g followed by 5.7 g after 5 min). The reaction was complete after 20 min (the progress was monitored by ¹H NMR in CDCl₃) and quenched upon vigorous stirring with 50 mL of mixed aqueous solution of 5% NaHCO₃ and 5% Na₂S₂O₃. The mixture was extracted with TBME (2 × 200 mL) and the combined organic layers were washed with 50 mL of sat. aq. NaHCO₃, dried over MgSO₄, filtered and concentrated *in vacuo.* The aldehyde was used without purification for the next step.
To a room temperature suspension of methyltriphenylphosphonium bromide (5.0 g, 14.02 mmol) in dry THF (40 mL) was added dropwise n-BuLi (2.5 M solution in hexanes, 5.1 mL, 12.74 mmol). The yellow suspension was stirred for 1 h at room temperature and then cooled to -78 °C. A solution of the crude aldehyde in dry THF (40 mL) was added dropwise over 10 min using additional 10 mL of dry THF to rinse the addition funnel. The reaction was left to warm up gradually to room temperature over 16 h. The reaction was quenched with 10 mL of aq. sat. NH₄Cl and extracted with AcOEt (3 × 100 mL). The combined organic layers were dried over MgSO₄, filtered and concentrated *in vacuo.* The residue was purified by column chromatography (SiO₂, petrol-acetone) to give the title alkene (0.94 g, 1.77 mmol, 28% yield over 2 steps) as a thin oil.
MS (ESI+) m/z 530 (M+H)⁺.

### Step 5: 7-O-benzoyl-D-glycero-6-O-benzoyl-3,4-di-O-benzyl-D-gluco-2-deoxy-2-phthalimido-1-β-deoxy-thioethyl-heptopyranoside

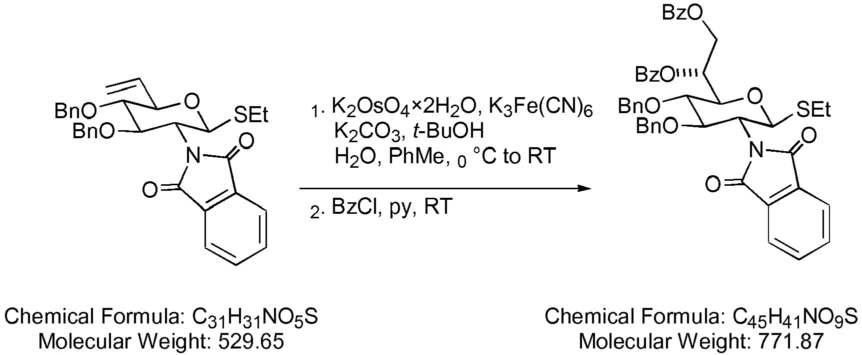

To a solution of K₂OsO₄·2H₂O (46 mg, 0.12 mmol), K₂CO₃ (1.78 g, 12.87 mmol) and K₃Fe(CN)₆ (3.83 g, 11.62 mmol) in H₂O (26 mL) and *t*-BuOH (26 mL) at 0 °C was added dropwise a solution of previously prepared alkene (2.20 g, 4.15 mmol) in toluene (26 mL). The reaction was allowed to gradually warm up to room temperature overnight (TLC system petrol:acetone 7:3), then Na₂SO₃ (8 g) added and the reaction stirred for 15 min at room temperature. The mixture was diluted with H₂O (50 mL) and extracted with TBME (250 mL), then AcOEt (2 × 100 mL). The combined organic layers were washed with brine (100 mL), dried over MgSO₄, filtered and concentrated in *vacuo.* ¹H NMR analysis of the crude mixture showed approximately a 3:1 ratio of (*R*):(*S*)-C6 epimers. The residue was dissolved in pyridine (15 mL) and benzoyl chloride (1.93 mL, 16.6 mmol) was added dropwise at room temperature. After 3 h the reaction was complete (TLC system CH₂Cl₂ 100%), quenched with H₂O (20 mL), diluted with CH₂Cl₂ (200 mL) and washed with 2M HCl (100 mL). The aqueous layer was extracted with CH₂Cl₂ (100 mL) and the combined organic layers were washed with 100 mL of sat. aq. NaHCO₃, dried over MgSO₄, filtered and concentrated *in vacuo.* The crude material was purified on high performance silica gel (gradient of 70 to 80% of CH₂Cl₂--petrol) to give 1.85 g of the major title epimer (viscous oil), 0.35 g of minor epimer (viscous oil) and 0.41 g of mixed fractions (total yield 2.61 g, 81% over 2 steps).

MS (ESI+) m/z 772 (M+H)⁺.

### Step 6: 7-O-benzoyl-D-glycero-6-O-benzoyl-3,4-di-O-benzyl-D-gluco-2-deoxy-2-phthalimido-1-β-acetyl-heptopyranoside

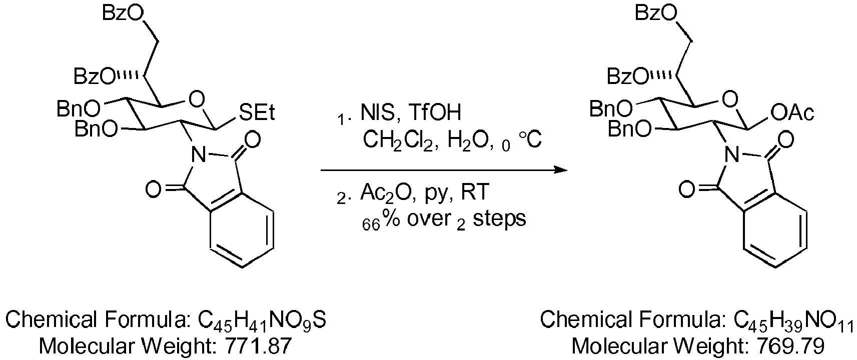

To a solution of previously prepared compound (587 mg, 0.76 mmol) in CH₂Cl₂ (25 mL) at 0 °C was added H₂O (1 mL) followed by NIS (206 mg, 0.915 mmol) and TfOH (16 µL). The mixture was stirred vigorously for 1 h, then diluted with CH₂Cl₂ (50 mL) and washed with a mixed aqueous solution of 5% NaHCO₃ and 5% Na₂S₂O₃ (50 mL). The aqueous layer was extracted with CH₂Cl₂ (3 × 50 mL) and the combined organic layers were dried over MgSO₄, filtered and concentrated *in vacuo.* The resulting crude syrup was dissolved in pyridine (15 mL) and Ac₂O (3 mL) was added dropwise at room temperature. After 2 h MeOH (20 mL) was added and the mixture was concentrated *in vacuo* to ∼50% volume, then diluted with CH₂Cl₂ (250 mL) and washed with 25% aqueous citric acid (100 mL) followed by brine (100 mL). The organic layer was dried over MgSO₄, filtered and concentrated *in vacuo.* The residue was purified by column chromatography (SiO₂, petrol-acetone) to give the title compound as a single anomer (viscous oil, 388 mg, 0.50 mmol, 66% over 2 steps).
MS (ESI+) m/z 770 (M+H)⁺.

### Step 7: 7-O-benzoyl-D-glycero-6-O-benzoyl-3,4-di-O-benzyl-D-gluco-2-deoxy-2-phthalimido-1-deoxy-heptopyranoside

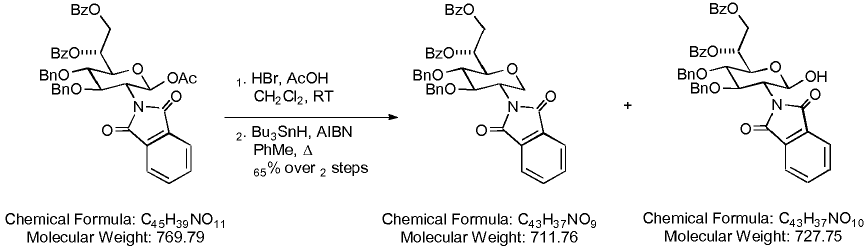

To a solution of previously prepared compound (438 mg, 0.569 mmol) in CH₂Cl₂ (20 mL) at 0 °C was added a solution of HBr (33% in AcOH, 0.28 mL), the cooling bath was removed and the reaction allowed to stir at room temperature for 2 h, then a further portion of HBr (33% in AcOH, 0.14 mL) was added at room temperature. After 1 h the mixture was diluted with CH₂Cl₂ (100 mL) and quenched with sat. aq. NaHCO₃ (50 mL) upon vigorous stirring. The aqueous layer was extracted with CH₂Cl₂ (3 × 50 mL) and the combined organic layers were dried over Na₂SO₄, filtered and concentrated *in vacuo.* The resulting yellow oil was co-evaporated with toluene (30 mL) under high vacuum for 20 min. The residue was dissolved in toluene (20 mL) and AIBN added (25 mg) followed by Bu₃SnH (0.276 mL, 1.024 mmol). Dry argon gas was bubbled through the solution for 10 min. The mixture was refluxed for 4 h then allowed to stir for 12 h at room temperature. The reaction was diluted with TBME (100 mL) then poured onto 5% aqueous solution of KF (80 mL) and stirred vigorously for 15 min. The aqueous layer was extracted with TBME (3 × 50 mL) and the combined organic layers were dried over MgSO₄, filtered and concentrated *in vacuo.* Purification by column chromatography (SiO₂, petrol-acetone) afforded the title 1-deoxy derivative (262 mg, 0.368 mmol, 65%) as a viscous oil and the hemi-acetal side-product (from bromide hydrolysis) (60 mg, 0.082 mmol, 14%).
MS (ESI+) m/z 712 (M+H)⁺.

### Step 8: D-glycero-3,4-di-O-benzyl-D-gluco-2-deoxy-2-benzyloxycarbonylamino-1-deoxy-heptopyranoside

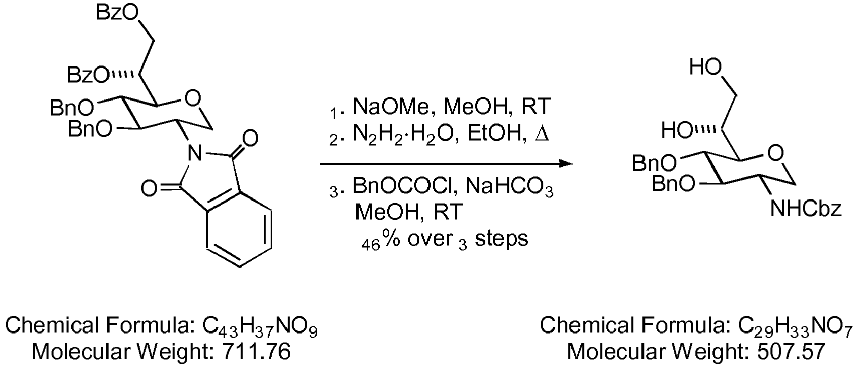

To a room temperature solution of previously prepared compound (240 mg, 0.337 mmol) in CH₂Cl₂ (4 mL) and MeOH (10 mL) was added a 25% solution of NaOMe in MeOH to pH 12. The reaction was complete within 2 h (TLC petrol-acetone 7:3) and was neutralised with Amberlyst® acidic resin, filtered and concentrated *in vacuo.* The residue was dissolved in EtOH (absolute, 15 mL) and hydrazine monohydrate (1.60 mL) was added at room temperature. The reaction was refluxed for 2 h, then concentrated *in vacuo* and co-evaporated with PhMe-MeOH 1:1 mixture (3 × 50 mL). The crude mixture was dissolved in MeOH (20 mL) and NaHCO₃ (200 mg, 2.381 mmol) was added followed by benzyl chloroformate (0.100 mL, 0.701 mmol) at room temperature. The mixture was stirred vigorously for 16 h then concentrated *in vacuo* and purified by column chromatography (SiO₂, toluene-acetone) to give the title compound as an off-white solid (79 mg, 0.156 mmol, 46% over 3 steps).
MS (ESI+) m/z 508 (M+H)⁺.

### Step 9: 6,7-di-O-tert-butyldimethylsilyl-D-glycero-3,4-di-O-benzyl-D-gluco-2-deoxy-2-benzyloxycarbonylamino-1-deoxy-heptopyranoside

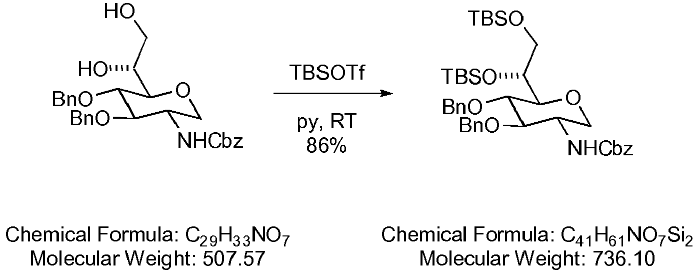

To a solution of previously prepared diol (79 mg, 0.156 mmol) in dry pyridine (5 mL) at 0 °C was added dropwise TBSOTf (0.357 mL, 1.556 mmol). The cooling bath was removed after 10 minutes and the mixture allowed to stir at room temperature. After 30 minutes the reaction was quenched with MeOH (5 mL) and evaporated to dryness. The residue was dissolved in CH₂Cl₂ (50 mL) and washed with 25% aqueous citric acid (15 mL). The aqueous layer was extracted with CH₂Cl₂ (30 mL) and the combined organic layers were washed with 15 mL of sat. aq. NaHCO₃, dried over MgSO₄, filtered and concentrated. The residue was purified by column chromatography (SiO₂, petrol-acetone) to give the title compound as viscous oil (98 mg, 0.133 mmol, 86%).
MS (ESI+) m/z 737 (M+H)⁺.

### Step 10: 6-O-tert-butyldimethylsilyl-D-glycero-3,4-di-O-benzyl-D-gluco-2-deoxy-2-benzyloxycarbonylamino-1-deoxy-heptopyranoside

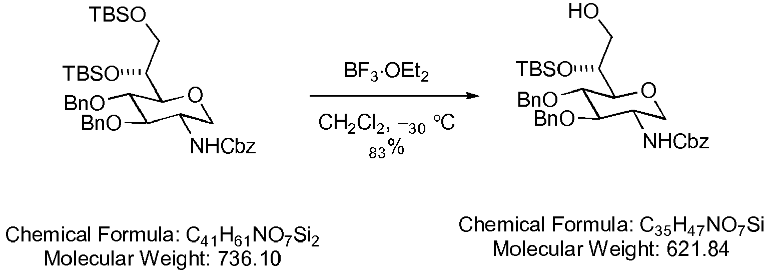

To a solution of previously prepared compound (40 mg, 0.054 mmol) in dry CH₂Cl₂ (5 mL) at -40 °C was added dropwise BF₃·OEt₂ (0.030 mL, 0.243 mmol). The progress of the reaction was monitored by TLC (petrol-acetone, 7:3). After 2 h the reaction was quenched with NH₃ and concentrated *in vacuo* at 35 °C. Purification by column chromatography (SiO₂, petrol-acetone) afforded the title compound (28 mg, 0.045 mmol, 83%) as a colorless oil.
MS (ESI+) m/z 622 (M+H)⁺.

### Step 11: 7-O-dibenzylphosphoryl-6-O-tert-butyldimethylsilyl-D-glycero-3,4-di-O-benzyl-D-gluco-2-deoxy-2-benzyloxycarbonylamino-1-deoxy-heptopyranoside

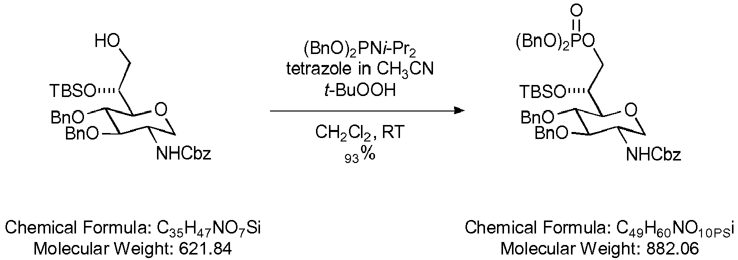

To a room temperature solution of previously prepared compound (56 mg, 0.090 mmol) in CH₂Cl₂ (10 mL) was added dropwise bisbenzyloxy-N,N-diisopropylaminophosphine (0.091 mL, 0.270 mmol) followed by tetrazole solution (0.45 M solution in CH₃CN, 0.721 mL, 0.324 mmol). After 1 h an additional portion of bisbenzyloxy-N,N-diisopropylaminophosphine (0.091 mL, 0.270 mmol) was charged followed by additional tetrazole solution (0.721 mL, 0.324 mmol). After 1.5 h a final portion of bisbenzyloxy-N,N-diisopropylaminophosphine (0.045 mL, 0.134 mmol) followed by tetrazole solution (0.360 mL, 0.162 mmol). The starting material was consumed within 1 h whereupon the reaction was cooled to 0 °C and *t*-BuOOH (80% solution in di-tert-butyl peroxide-H₂O 3:2, 0.056 mL, approx. 0.45 mmol) was added. The mixture was allowed to warm up to room temperature gradually and stirred at ambient temperature overnight. The mixture was diluted with CH₂Cl₂ (50 mL) and washed with a 1:1 mixture of 10% aq. solution of Na₂S₂O₃ and sat. aq. NaHCO₃ (20 mL). The aqueous layer was extracted with CH₂Cl₂ (2 × 25 mL) and the combined organic layers were dried over MgSO₄, filtered and concentrated. The residue was purified by column chromatography (SiO₂, toluene-acetone) to give the title compound (74 mg, 0.084 mmol, 93%).
MS (ESI+) m/z 883 (M+H)⁺.

### Step 12: 7-O-dibenzylphosphoryl-D-glycero-3,4-di-O-benzyl-D-gluco-2-deoxy-2-benzyloxycarbonylamino-1-deoxy-heptopyranoside

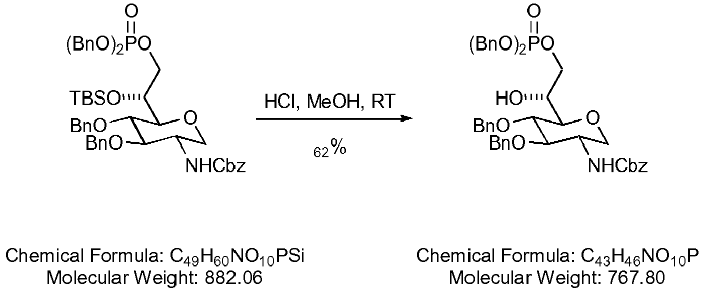

To a room temperature solution of previously prepared compound (67 mg, 0.076 mmol) in MeOH (5 mL) a 1.25 M solution of HCl in MeOH was added to pH 4/5 and the reaction was allowed to stir at room temperature for 16 h. The reaction was neutralised with sat. aq. NaHCO₃ and concentrated *in vacuo.* The solid residue was dissolved with CH₂Cl₂ (50 mL) and washed with H₂O (10 mL). The organic layer was dried over MgSO₄, filtered and concentrated *in vacuo.* Purification by column chromatography (SiO₂, toluene-acetone) furnished the title compound (36 mg, 0.047 mmol, 62%) as an off-white solid.
MS (ESI+) m/z 768 (M+H)⁺.

### Step 13: 7-O-phosphate-D-glycero-D-gluco-2-deoxy-2-amino-1-deoxy-heptopyranoside hydrochloride

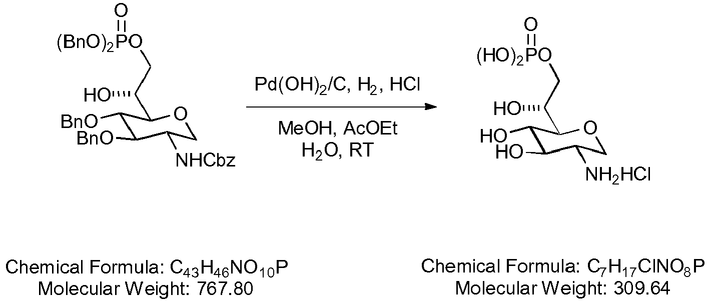

The previously prepared sugar (33 mg, 0.0430 mmol) was dissolved in MeOH (3 mL) and 1.25 M HCl in MeOH was added to pH 4/5 followed by Pd(OH)₂/C. The reaction flask was evacuated and flushed three times with hydrogen gas. The reaction was kept under a hydrogen balloon at room temperature for 16 h, then 1 mL of H₂O added and reaction stirred for further 1h at room temperature under hydrogen balloon. The palladium catalyst was filtered and rinsed with MeOH (40 mL) and H₂O (50 mL). The solvents were removed *in vacuo* and the residue co-evaporated with H₂O (2 × 10 mL), then purified by reverse phase chromatography (C18, 0.5 g cartridge) using H₂O as eluent. The product was freeze-dried to give 13.8 mg of the final product (0.045 mmol, quantitative).
MS (ESI+) m/z 310 (M+H)⁺.
¹H NMR (400 MHz, D₂O): δ = 4.15-4.10 (m, 2H, C1*H*_{A}H_{B} and C6H), 4.04-3.99 (m, 1H, C7*H*_{A}H_{B}), 3.92-3.85 (m, 1H, C7H_{A}*H*_{B}), 3.60-3.53 (m, 2H, C3H and C4H), 3.49-4.42 (m, 2H, C1H_{A}*H*_{B} and C5H), 3.30-3.22 (m, 1H, C2H).
¹³C NMR (100 MHz, D₂O): δ = 80.8 (C5H), 73.8 (C3H), 70.2 (d, J 8.6, C6H), 70.1 (C4H), 65.7 (C1H₂), 61.6 (d, J 5.5, C7H₂), 51.2 (C2H).
³¹P NMR (162 MHz, D₂O): δ = 0.50.

### Example 2: 7-O-phosphate-D-glycero-D-gluco-2-deoxy-2-amino-heptopyranoside hydrochloride

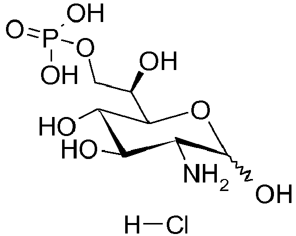

### Step 1: 7-O-benzoyl-D-glycero-6-O-benzoyl-3,4-di-O-benzyl-D-gluco-2-deoxy-2-phthalimido-1-β-O-benzyl-heptopyranoside

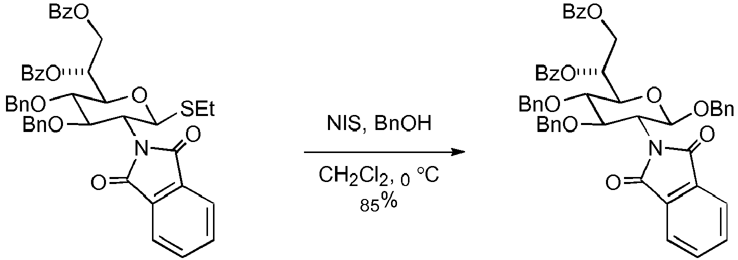

To a solution of 7-O-benzoyl-D-*glycero*-6-O-benzoyl-3,4-di-O-benzyl-D-*gluco*-2-deoxy-2-phthalimido-1-β-deoxy-thioethyl-heptopyranoside prepared as described in example 1 step 5 (1.13 g, 1.46 mmol) in CH₂Cl₂ (25 mL) were added 4Å molecular sieves (5 g, ground, activated) and BnOH (0.61 mL, 5.89 mmol) and the resulting suspension was stirred at room temperature for 30 min. NIS (0.66 g, 2.94 mmol) was added in one portion and the reaction mixture was cooled to 0 °C. After 15 minutes TMSOTf (0.29 mL, 1.60 mmol) was added dropwise and the reaction stirred at 0 °C for 30 min, then quenched with Et₃N (0.50 mL), filtered and concentrated *in vacuo* at 35 °C. The residue was purified by column chromatography (SiO₂, petrol-acetone) to give the title compound as a viscous oil (1.01 g, 85% yield).
MS (ESI+) m/z 818 (M+H)⁺.

### Step 2: D-glycero-3,4-di-O-benzyl-D-gluco-2-deoxy-2-phthalimido-1-β-O-benzyl-heptopyranoside

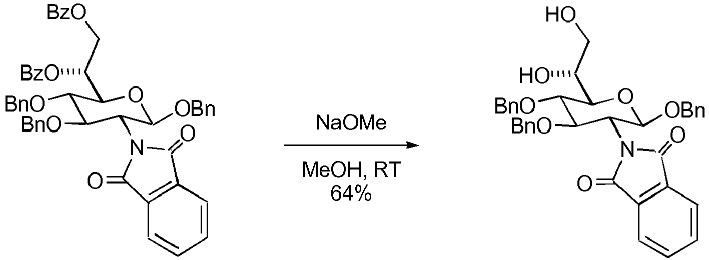

To a solution of the previously prepared compound (1.01g, 1.23 mmol) in MeOH (50 mL) at room temperature was added a 25% solution of NaOMe in MeOH to pH 12. The reaction was complete within 2 h (TLC petrol-acetone 7:3) and was neutralised with Amberlyst®15 acidic resin, filtered and concentrated *in vacuo.* The residue was purified by column chromatography (SiO₂, petrol-acetone) to give the title diol as viscous oil (0.48 g, 0.79 mmol, 64%).
MS (ESI+) m/z 610 (M+H)⁺.

### Step 3: D-glycero-3,4-di-O-benzyl-D-gluco-2-deoxy-2-benzyloxycarbonylamino-1-β-O-benzyl-heptopyranoside

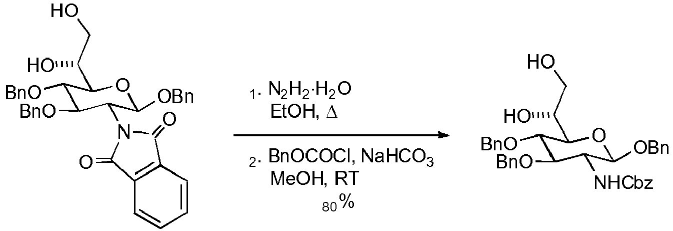

To a solution of previously prepared diol (320 mg, 0.525 mmol) in EtOH (18 mL) at room temperature was added hydrazine monohydrate (2.50 mL) and the reaction was refluxed for 2.5 h. The mixture was then concentrated *in vacuo* and co-evaporated with toluene (4 × 50 mL) and MeOH (4 × 50 mL). The crude mixture was dissolved in MeOH (25 mL) and NaHCO₃ (220 mg, 2.625 mmol) was added followed by benzyl chloroformate (0.131 mL, 0.9186 mmol) at room temperature. The mixture was stirred vigorously and after 16 h additional NaHCO₃ (220 mg, 2.6245 mmol) and benzyl chloroformate (0.131 mL, 0.9186 mmol) were added. The reaction was complete within 1 h, concentrated *in vacuo* and purified by column chromatography (SiO₂, toluene-acetone) to give the protected title amine as a white solid (0.213 g, 0.420 mmol, 80%).
MS (ESI+) m/z 614 (M+H)⁺.

### Step 4: 6,7-bis(O-tert-butyldimethylsilyl)-D-glycero-3,4-di-O-benzyl-D-gluco-2-deoxy-2- benzyloxycarbonylamino-1-β-O-benzyl-heptopyranoside

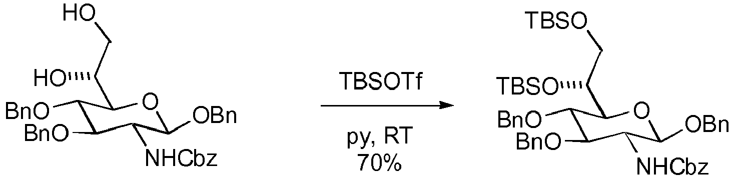

To a room temperature solution of previously prepared diol (131 mg, 0.214 mmol) in dry pyridine (10 mL) was added dropwise TBSOTf (0.492 mL, 2.144 mmol). After 16 h the reaction mixture was diluted with CH₂Cl₂ (100 mL) and washed with 2M HCl (25 mL). The aqueous layer was extracted with CH₂Cl₂ (50 mL) and the combined organic layers were washed with 25 mL of sat. aq. NaHCO₃, dried over MgSO₄, filtered and concentrated. The residue was purified by column chromatography (SiO₂, petrol-acetone) to give the fully protected title compound as viscous oil (126 mg, 0.150 mmol, 70%).
MS (ESI+) m/z 843 (M+H)⁺.

### Step 5: 6-O-tert-butyldimethylsilyl-D-glycero-3,4-di-O-benzyl-D-gluco-2-deoxy-2- benzyloxycarbonylamino-1-β-O-benzyl-heptopyranoside

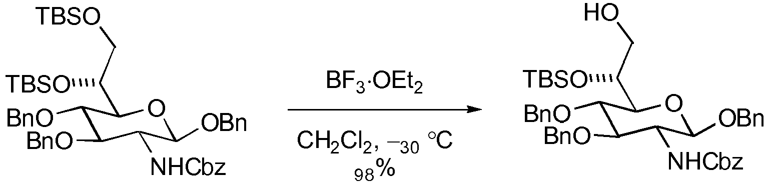

To a solution of previously prepared compound (110 mg, 0.131 mmol) in dry CH₂Cl₂ (10 mL) at -30 °C was added dropwise BF₃·OEt₂ (0.081 mL, 0.653 mmol). The progress of the reaction was monitored by TLC (petrol-acetone, 7:3). After 3 h the reaction was quenched with aq. sat. NaHCO₃ and concentrated *in vacuo* at 35 °C. The residue was dissolved with CH₂Cl₂ (50 mL) and washed with aq. sat. NaHCO₃ (25 mL). The aqueous layer was extracted with CH₂Cl₂ (25 mL) and the combined organic layers were dried over MgSO₄, filtered and concentrated *in vacuo.* Purification by column chromatography (SiO₂, petrol-acetone) afforded the title compound (93 mg, 0.128 mmol, 98%) as viscous oil.
MS (ESI+) m/z 728 (M+H)⁺.

### Step 6: 7-O-dibenzylphosphoryl-D-glycero-3,4-di-O-benzyl-D-gluco-2-deoxy-2- benzyloxycarbonylamino-1-β-O-benzyl-heptopyranoside

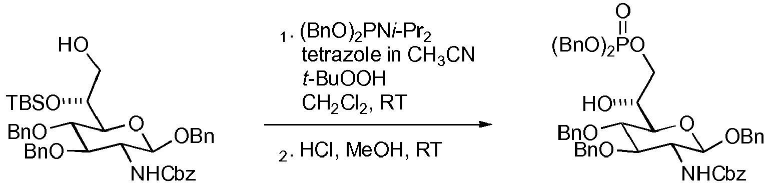

To a room temperature solution of previously prepared compound (93 mg, 0.1278 mmol) in CH₂Cl₂ (15 mL) was added dropwise bisbenzyloxy-N,N-diisopropylaminophosphine (0.129 mL, 0.3833 mmol) followed by tetrazole solution (0.45M solution in CH₃CN, 1.02 mL, 0.4599 mmol). The starting material was consumed within 2.5 h whereupon the reaction was cooled to 0 °C and *t*-BuOOH (80% solution in di-tert-butyl peroxide-H₂O 3:2, 0.064 mL, approx. 0.64 mmol) was added. The mixture was allowed to warm up to room temperature over 3 h then diluted with CH₂Cl₂ (100 mL) and washed with 10% aq. solution of Na₂S₂O₃ (30 mL). The aqueous layer was extracted with CH₂Cl₂ (30 mL) and the combined organic layers were washed with sat. aq. NaHCO₃ (30 mL), dried over MgSO₄, filtered and concentrated. The residue was purified through a short silica plug (toluene-acetone) to give 177 mg of phosphorylated sugar intermediate contaminated with bisbenzyloxy-N,N-diisopropylaminophosphine residue.
The residue was dissolved in MeOH (15 mL) and 1.25 M HCl in MeOH was added to pH 4/5 and reaction was allowed to stir at room temperature for 16 h. The reaction was neutralised with sat. aq. NaHCO₃ and concentrated *in vacuo.* The solid residue was dissolved with CH₂Cl₂ (100 mL) and washed with H₂O (25 mL). The aqueous layer was extracted with CH₂Cl₂ (2 × 30 mL), the combined organic layers were dried over MgSO₄, filtered and concentrated *in vacuo.* Purification by column chromatography (SiO₂, toluene-acetone) gave 40 mg of the title compound (0.046 mmol, 38% over 2 steps, about 95% purity as judged by ¹H NMR) and 30 mg of TBS protected starting material contaminated with residual bisbenzyloxy-N,N-diisopropylaminophosphine. The 40 mg of product was repurified by column (SiO₂ toluene-acetone) to give 35 mg of the title compound as viscous oil.
MS (ESI+) m/z 874 (M+H)⁺.

### Step 7: 7-O-phosphate-D-glycero-D-gluco-2-deoxy-2-amino-heptopyranoside hydrochloride

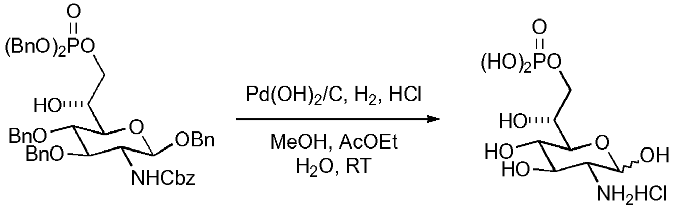

The previously prepared phosphorylated sugar (35 mg, 0.040 mmol) was dissolved in MeOH (2 mL) and AcOEt (2 mL) and 1.25 M HCl in MeOH was added to pH 4/5. Pd(OH)₂/C was added under argon and the reaction flask was evacuated and flushed three times with hydrogen gas. The reaction was kept under a hydrogen balloon at room temperature for 16 h, then 3 mL of H₂O added and reaction stirred for further 5 h at room temperature under hydrogen balloon. The palladium catalyst was filtered and the solvent was concentrated *in vacuo,* the residue was co-evaporated with MeOH (3 × 30 mL), then purified on a reverse phase cartridge (C18, 0.5 g cartridge) using H₂O as eluent. The product was freeze-dried to give 17.6 mg of the final title product.
MS (ESI+) m/z 290 (M+H)⁺.
α to β anomers ratio is 1 : 0.6
¹H NMR (400 MHz, D₂O): δ = 5.39 (d, 1H, *J* 3.2, α-C1H), 4.87 (d, 0.6H, *J* 8.4, β-C1H), 4.16-4.12 (m, 1H and 0.6H, α-C6H and β-C6H), 4.06-4.01 (m, 1H and 0.6H, α-C7*H_{A}*H_{B} and β-C7*H_{A}*H_{B}), 3.96-3.85 (m, 2x1H and 1x0.6H, α-C7H_{A}*H_{B}*, α-C5H and β-C7HA*H_{B}*), 3.83 (t, 1H, *J* 10.2, α-C3H), 3.62-3.56 (m, 1×1H and 3×0.6H, α-C4H and β-C3H, β-C4H, β-C5H), 3.28 (dd, 1H, *J* 10.4, 3.1, α-C2H), 2.98 (t, 0.6H, *J* 8.4, β-C2H).
¹³C NMR (100 MHz, D₂O): δ = 93.0 (β-C1H), 89.2 (α-C1H), 76.1 (β-C5H), 72.2 (β-C3H), 71.5 (α-C5H), 70.4, 70.3, 70.3, 70.2 (α-C6H, β-C6H, α-C4H and β-C4H), 69.9 (α-C3H), 65.6 (d, *J* 5.0, α-C7H₂), 65.3 (d, *J* 5.2, β-C7H2), 56.6 (β-C2H), 54.1 (α-C2H).
³¹P NMR (162 MHz, D₂O): δ = 0.55.

### Examples of pharmaceutical compositions

Solutions for IV administration have been prepared by diluting the compound of example 2 at 1-20 mg/mL in physiological serum (0.9% sodium chloride in non-pyrogenic water) or in glucose solution (1-5% glucose in non-pyrogenic water), with and without addition of erythromycin (1-5 mg/mL).

Other solutions for IV administration have been prepared by diluting 500 mg of compound of example 2 in 100 mL of physiological serum (0.9% sodium chloride in non-pyrogenic water) or of glucose solution (5% glucose in non-pyrogenic water).

### Pharmacological study of the compounds of the invention

### Inhibition of the enzymatic activity of GmhA (luminescent assay):

The assay buffer "AB" contained 50 mM Hepes pH7.5, 1 mM MnCl₂, 25 mM KCl, 0.012% Triton-X100 and 1mm dithiothreitol (DTT) and 0.1µM Myelin basic protein (MBP). The following components were added in a white polystyrene Costar plate up to a final volume of 30µL: 10µL inhibitor dissolved in DMSO/water 50/50, and 20µL GmhA of E. *coli* in AB. After 30min of pre-incubation at room temperature, 30µL of Substrates mix in AB were added in each well to a final volume of 60µL. This reaction mixture was then composed of 2nM GmhA, 3µM sedoheptulose-7-phosphate (Sigma), 3µM ATP (Sigma) and 50nM HldE of E. coli in assay buffer. After 30min of incubation at room temperature, 100µL of the revelation mix were added to a final volume of 160µL, including the following constituents at the respective final concentrations: 10000 light units/ml luciferase (Sigma), 20µM D-luciferin (Sigma), 100µM N-acetylcysteamine (Aldrich). Luminescence intensity was immediately measured on Luminoskan (Thermofischer) and converted into inhibition percentages. For IC50 determinations, the inhibitor was tested at 6 to 10 different concentrations, and the related inhibitions were fitted to a classical langmuir equilibrium model using XLFIT (IDBS).

### Inhibition of the enzymatic activity of HldE-K (luminescent assay on kinase activity):

The assay buffer "AB" contained 50 mM Hepes pH7.5, 1 mM MnCl₂, 25 mM KCl, 0.012% Triton-X100 and 1mm dithiothreitol (DTT) and 0.1µM Myelin basic protein (MBP). The following components were added in a white polystyrene Costar plate up to a final volume of 30µL: 10µL inhibitor dissolved in DMSO/water 50/50, and 20µL HldE of E. *coli* in AB. After 30min of pre-incubation at room temperature, 30µL of Substrates mix in AB were added in each well to a final volume of 60µL. This reaction mixture was then composed of 3nM HldE, 0.2µM β-heptose-7-phosphate (custom synthesis) and 0.2µM ATP (Sigma) in assay buffer. After 30min of incubation at room temperature, 200µL of the revelation mix were added to a final volume of 260µL, including the following constituents at the respective final concentrations: 5000 light units/ml luciferase (Sigma), 30µM D-luciferin (Sigma), 100µM N-acetylcysteamine (Aldrich). Luminescence intensity was immediately measured on Luminoskan (Thermofischer) and converted into inhibition percentages. For IC50 determinations, the inhibitor was tested at 6 to 10 different concentrations, and the related inhibitions were fitted to a classical Langmuir equilibrium model using XLFIT (IDBS).

### Inhibition of E. coli C7 (018:K1:H7) LPS biosynthesis:

*Principle: E. coli* C7 (018:K1:H7) is a Newborm Meningitidis E. *coli* (NMEC) strain which displays a typical LPS made of Lipid A successively branched with the inner and outer core oligosaccharides, and finally with the O-antigen repeats. The inner core contains several heptose residues. An inhibitor of the LPS heptosylation pathway should therefore reduce dramatically the size of LPS from full-length to the so-called 'Re-LPS' limited to lipid A branched with 2 Kdo residues. A simple way of monitoring LPS size and composition consists in running LPS gel electrophoresis (Figure 1): a wild type E. *coli* strain displays several bands including those for full and core LPS but none for Re-LPS. On the contrary, a delta-hldE mutant defective for LPS-heptosylation biosynthesis displays only the Re-LPS band.

*Bacterial culture:* The effect of heptosylation inhibitors on *E. coli* LPS was assessed as described below. The compounds to be tested were prepared in deionised water/DMSO (50/50) solutions and added (25µL) in sterile culture microtubes. The strain used in this study was *E. coli* C7 (018:K1:H7). The bacteria were isolated on tryptic soy agar (TSA) over-night. Isolated colonies were cultured in 10ml of Luria-Bertani medium (LB) at 37°C up to an optical density of typically 0.15. These exponentially growing bacteria were finally diluted to 5e5 cfu/ml and added in each well (225µL) for incubation with the compounds at 37°C for approximately 5 hours, up to an optical density of ≈0.2-0.4. Some test compounds e.g. phospho-sugars required Glucose-6-Phosphate (G6P, from Sigma) to be added in the culture medium in order to activate their active transport into the bacterial cytosol via the phospho-sugar transporter UhpT. This was achieved by adding in the culture tube 2.5µL of a 10mM water solution of G6P (100µM final concentration).

*LPS extraction:* Bacterial cultures were normalized via OD determination, pelleted and washed with 1ml Phosphate-Buffer-Saline (PBS). The pellets were then denatured for 10min at 95-100°C in 50µl of Sodium-Dodecyl-Sulfate 0.2%(SDS), beta-mercaptoethanol 1%, Glycerol 36%, Tris pH7.4 30mM and bromophenol blue 0.001%. Samples were cooled down to room temperature, supplemented with 1.5µl of proteinase K at 20mg/ml, incubated for 1H at 55°C and centrifuged for 30min at 13000rpm at 25°C. The resulting supernatant, containing LPS was finally analysed by SDS-PAGE electrophoresis.

*LPS SDS-PAGE electrophoresis*: Polyacrylamide gels (16% / 4% acrylamide for separation and concentration respectively) were prepared, loaded with 8µl of LPS extracts and migrated.

*Silver staining*: Gels were incubated overnight in 5% acetic acid/40% ethanol/deionised water, treated by 1% periodic acid/5% acetic acid for 15min, washed 4 times for 10min in deionised water and finally incubated for 18min in the dark in a silver nitrate solution composed of 56ml NaOH 0.1N, 4ml ammoniac 33%, 45ml AgN03 5% (Tsai and Frasch) and 195 ml deionised water. Gels were then washed extensively in deionised water for 30min and incubated for 10-15min (up to LPS bands apparition) in the revelation mix composed of 300ml deionised water, 300µl formaldehyde 36.5% (Fluka) and 100µl citric acid 2.3M. The revelation was stopped by incubating the gels in acetic acid 10% for 5min. Gels were finally washed in deionised water, numerized with a Samsung PL51 camera and analysed by ImageJ software. The percentage of inhibition of LPS heptosylation was defined as the relative area of the Re-LPS band compared to the cumulated areas of Re-LPS and Core-LPS bands.

### Inhibitory activities of selected compounds:

Compounds described in examples **1** and **2** display IC50 values < 100 µM on GmhA.

Compounds described in examples **1** and **2** display IC50 values < 100 µM on HldE-K.

Compound described in example **2** displays in the presence of 100 µM G6P at least 50% inhibition of E. coli C7 LPS heptosylation at concentrations < 1 mM.

Such compound did not kill the bacteria at 1 mM, and was inactive in reducing LPS heptosylation in absence of G6P. It is therefore reaching its target in the cytosol specifically via the phospho-sugar transporter UhpT.
We provide here a novel non-antibiotic compound active on LPS heptosylation targets while also demonstrating the ability to reach and inhibit these targets in the cytosol. This resulted in significant inhibition of LPS heptosylation on clinically relevant Gram-negative bacteria.

Data obtained on bacteria lacking heptoses show that such compounds have the potential to induce a high sensitivity of Gram-negative bacteria to the host complement or to detergents and hydrophobic antibiotics They have therefore the potential to prevent or inhibit bacterial infection alone or in combination with an antibiotic, antivirulent or immunostimulating drug (Annu. Rev. Biochem. 2002, 635).

Such inhibitors provide a novel way to treat or prevent bloodstream infections caused by pathogenic Gram negative bacteria, without affecting the commensal flora and with less selective pressure than conventional antibacterial agents.

## Claims

1. The compounds of general formula (I) 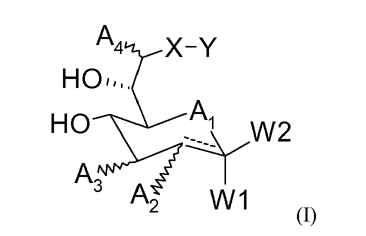 wherein,
- the dotted line represents a possible second bond;
- X is CH₂, CHF, CF₂, CHOH, O, S or NH;
- Y is P(O)(ORₐ)(OR_{b}) or P(O)(ORₐ)(NRₐR_{b});
- A₁ is O, S, NRₐ, N(CO)Rₐ, N(SO)Rₐ or N(SO₂)Rₐ;
- A₂ and A₃, identical or different, are H, F, NR_{c}R_{d}, NR_{c}(CO)R_{d}, NR_{c}(SO)R_{d}, NR_{c}(SO₂)R_{d} or OR_{c}, and A₂ may also be an oxime NOR_{c}, in which case the dotted line doesn't represent a bond;
- A₄ is H, F or OH;
- W1 and W2 identical or different, are selected from the group consisting of H, F, CN, (C₁-C₆) alkyl, (C₁-C₆) fluoroalkyl, (C₂-C₆) alkenyl, (C₂-C₆) alkynyl, OR_{c}, (CO)OR_{c}, (CO)NR_{c}R_{d}, NR_{c}R_{d}, O(CO)R_{c}, O(CO)OR_{c}, O(CO)NR_{c}R_{d}, S(O)R_{c}, S(O)₂R_{c}, (C₁-C₆)alkyl-OR_{c}, (C₁-C₆)alkyl-NR_{c}R_{d}, (C₁-C₆)alkyl-(CO)OR_{c}, (C₁-C₆)alkyl-(CO)NR_{c}R_{d}, (C₁-C₆)alkyl-O(CO)R_{c}, (C₁-C₆)alkyl-O(CO)OR_{c}, (C₁-C₆)alkyl-O(CO)NR_{c}R_{d}, (C₁-C₆)alkyl-S(O)R_{c} or (C₁-C₆)alkyl-S(O)₂R_{c}, NR_{c}(CO)R_{d}, NR_{c}(CO)OR_{d}, NR_{c}(SO₂)R_{d};
or W1 and W2 form together a saturated 3-6 membered carbon cycle or heterocycle with 1 to 3 ring heteroatoms selected from N, O and S; W2 being absent when the dotted line represents a second bond;
- Rₐ and R_{b}, identical or different, are selected from the group consisting of H, (C₁-C₆)alkyl, C₁-C₆)fluoroalkyl, (C₁-C₆)alkyl-OH, (C₁-C₆)alkyl-O-(C₁-C₆) alkyl;
- R_{c} and R_{d}, identical or different, are selected from the group consisting of H, (C₁-C₆)alkyl, C₁-C₆)fluoroalkyl, (C₂-C₆)alkenyl, (C₂-C₆)alkynyl, (C₁-C₆)alkyl-OH, (C₁-C₆)alkyl-O-(C₁-C₆)alkyl, Ph, Het, CH₂Ph, CHMePh, CMe₂Ph, CH₂Het, CHMeHet, CMe₂Het, Het being a 4-6 membered saturated or unsaturated heterocycle with 1 to 3 ring heteroatoms selected from N, O and S and optionally substituted by a keto, alkyl, fluoroalkyl, OH, NH2, O-alkyl, NH-alkyl or N(dialkyl), the above alkyls being C₁-C₆;
with the proviso that when A₂ and A₃ are OH and A₄ is H then A₁ may not be O;
and to the exclusion of 3-Deoxy-D-altro-octulosonate 8-phosphate;
and their addition salts thereof with acids and bases.

2. The compounds of formula (I) and their salts, according to claim 1, wherein Y is P(O)(OH)₂.

3. The compounds of formula (I) and their salts, according to claim 1 or 2, wherein X is CH₂, CHF, CF₂, CHOH or O.

4. The compounds of formula (I) and their salts, according to any of claims 1 to 3, wherein A₁ is O.

5. The compounds of formula (I) and their salts, according to any of claims 1 to 4, wherein A₃ is selected from the group consisting of H, F, OR_{c} and NR_{c}R_{d}.

6. The compounds of formula (I) and their salts, according to any of claims 1 to 5, wherein W1 and W2, identical or different, are selected from the group consisting of H, (C₁-C₆)alkyl, (C₁-C₆)fluoroalkyl, OR_{c} and (C₁-C₆)alkyl-OR_{c}.

7. The compounds of formula (I) and their salts, according to any of claims 1 to 6, wherein R_{c} and R_{d} are selected from the group consisting of H, (C₁-C₆)alkyl, C₁-C₆)fluoroalkyl, (C₁-C₆)alkyl-OH, (C₁-C₆)alkyl-O-(C₁-C₆)alkyl and Het.

8. The compounds of formula (I) which names follow:
7-O-phosphate-D-*glycero*-D-*gluco*-2-deoxy-2-amino-1-deoxy-heptopyranoside,
7-O-phosphate-D-*glycero*-D-*gluco*-2-deoxy-2-amino-heptopyranoside,
and their salts, in particular their hydrochlorides.

9. A process for the preparation of compounds of formula (I) as defined in claim 1, comprising the step of reacting a compound of formula (V): 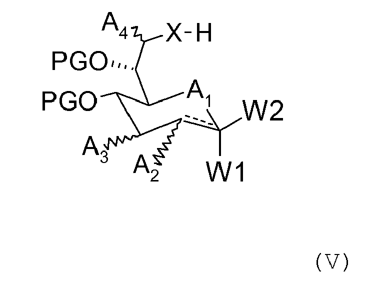 in which X, A₁, A₂, A₃, A₄, W1 and W2 are as defined in claim 1, the same being optionally protected by protecting groups, the two PG are Hydrogen or identical or different protecting groups, with a compound of formula Y-LG, in which Y is as defined in claim 1 and LG is a leaving group, the reaction being optionally followed by deprotection of PG to hydrogen.

10. A process for the preparation of compounds of formula (I) as defined in claim 1, comprising the step of reacting a compound of formula (VI): 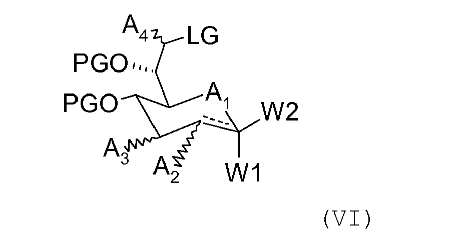 in which A₁, A₂, A₃, A₄, W1 and W2 are as defined in claim 1, the same being optionally protected by protecting groups, the two PG are Hydrogen or identical or different protecting groups and LG is a leaving group, with a compound of formula MeP(O)(ORₐ)(OR_{b}), MeP(O)(ORₐ)(NRₐR_{b}), CHFP(O)(ORₐ)(OR_{b}), CHFP(O)(ORₐ)(NRₐR_{b}), CF₂P(O)(ORₐ)(OR_{b}) or CF₂P(O)(ORₐ)(NRₐR_{b}), in the presence of a base, the reaction being optionally followed by deprotection of PG to hydrogen.

11. A process for the preparation of compounds of formula (I) as defined in claim 1, comprising the step of deprotecting a compound of formula (VII) or a salt thereof: 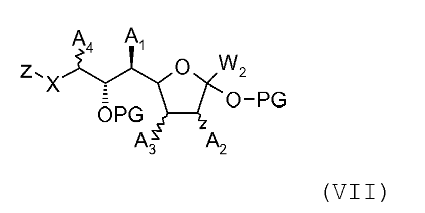 in which A₁, A₂, A₃, A₄, W2 and X are as defined in claim 1, the same being optionally protected by identical or different protecting groups PG, Z is Y, as defined in claim 1, or PG, and the groups PG are Hydrogen or identical or different protecting groups.

12. The compounds of general formula (I) and pharmaceutically acceptable addition salts thereof with acids and bases as defined in claim 1, for use as drugs.

13. The compounds of general formula (I) and pharmaceutically acceptable addition salts thereof with acids and bases as defined in any of claims 2 to 7, for use as drugs.

14. The compounds of general formula (I) and pharmaceutically acceptable addition salts thereof with acids and bases, as defined in claim 8, for use as drugs.

15. The compounds of general formula (I) and pharmaceutically acceptable addition salts thereof with acids and bases as defined in any of claims 1 to 8, for use as drugs for the prevention and/or therapeutical treatment of severe infections due to Gram-negative bacteria.

16. The compounds of general formula (I) and pharmaceutically acceptable addition salts thereof with acids and bases, as defined in any of claims 1 to 8, for use as drugs, in association with either an antibacterial, an antivirulence agent or a drug reinforcing the host innate immunity.

17. The compounds of general formula (I) and pharmaceutically acceptable addition salts thereof with acids and bases, as defined in any of claims 1 to 8, for use as drugs, in association with macrolides, streptogramins, pleuromutilins, FabI inhibitors, rifamycins, lipopeptides or GM-CSF.

18. The compounds of general formula (I) and pharmaceutically acceptable addition salts thereof with acids and bases, as defined in any of claims 1 to 8, for use in association with either an antibacterial, an antivirulence agent or a drug reinforcing the host innate immunity, as drugs for the prevention and/or therapeutical treatment of severe infections due to Gram-negative bacteria.

19. The pharmaceutical compositions containing as active principle a compound of general formula (I) or a pharmaceutically acceptable addition salt thereof with an acid or a base, as defined in any of claims 1 to 8.

20. Mixture or pharmaceutical association comprising as active principles a compound of formula (I) or a pharmaceutically acceptable addition salt thereof with an acid or a base, as defined in any of claims 1 to 8, and an antibacterial, an antivirulence agent or a drug reinforcing the host innate immunity.

21. Mixture or pharmaceutical association comprising as active principles a compound of formula (I) or a pharmaceutically acceptable addition salt thereof with an acid or a base, as defined in any of claims 1 to 8, and a macrolide, a streptogramin, a pleuromutilin, a FabI inhibitor, a rifamycin, a lipopeptide or a GM-CSF.
